# EUROPEAN PATENT SPECIFICATION

(11) **EP 2 783 662 B1**
(45) Date of publication and mention of the grant of the patent: **28.08.2019**
(21) Application number: 14000904.4
(22) Date of filing: 13.03.2014
(51) Int. Cl.: A61F 5/055

(54) **Conformable cervical collar**
Anpassbare Halskrause
Collier cervical adaptable

(30) Priority: 26.03.2013 IT MI20130109 U
(43) Date of publication of application: 01.10.2014
(73) Proprietor: Flamor S.r.l., 28060 San Pietro Mosezzo (NO) (IT)
(72) Inventor: Limontini, Flavio, 28060 San Pietro Mosezzo (NO) (IT)
(74) Representative: Valentini, Giuliano

(56) References cited:
- FR-A1- 2 815 245
- GB-A- 2 303 792
- US-A- 3 320 950
- US-A- 4 676 233

## Description

The present invention relates to a cervical collar and, in particular, a cervical stiff collar intended to be used in the first aid operations to prevent undesired bending, stretching or rotation movements of the neck. Such collars are applied to a patient in order to avoid potential damages to the cervical vertebrae which could result in permanent lesions or paralysis, if not even the patient death, as a consequence of the spinal nerve damaging.

Cervical collars of known type intended for the first aid operations generally include a body made of plastic material of the elastically flexible type, for example made of polyethylene, coated with a closed-cell expanded foam, such as for example EVA or the like. The collar body is suitably shaped for providing support and/or rest to the various body parts near the neck, for example chin, mandible, breastbone and nape.

Such collars generally show a starting flat shape including at its ends closing means, such as for example Velcro™ strips or the like. During patient application, the collar is suitably positioned behind the nape, then bended for completely wrapping the patient neck, and finally closed by using the closing means it is provided with. After the removal from the patient neck, the collar goes back to its original flat shape and can therefore be put away for a next use.

However, one of the main problems of such cervical collars is given by their encumbrance; in fact, when the collar is in its original flat shape, its overall length can also exceed 60 cm. These collars often have to be part of the emergency equipment which is carried in a backpack, for example over field military operations or interventions of the rescue personnel in sites confronting disasters.

One more problem of the cervical collars of known type is given by the complexity of use. For example, in some commercially supplied models, the portion supporting the chin (chin rest), is mobile to be fitted to patient size and shape; once the collar has been applied, the chin rest is moved to a suitable position and its movement is inhibited by suitable means which hold it in position. Therefore it appears that the application of such collars is rather complex, slow and potentially dangerous, especially taking into account that these operations often occur in adverse conditions and the time factor is often determinant for patient safety.

US 3320950 discloses a cervical collar according to the preamble of claim 1.

GB 2303792 discloses an orthopaedic neck support made of a shaped flexible foam with compressible areas in the chin and chest regions. A plurality of cuts are provided in the compressible areas in order to form fingers which make more compressible and/or flexible the foam in the chin and chest regions.

FR 2815245 discloses a cervical collar made of an outer sleeve of flexible material and an inner stiffening element.

It is also known by the patent n. US 4,676,233 a particular immobilizing means for fractured limbs, also known as "immobilizing splint", constituted by a strip of material that can be shaped and able to provide the stiffness required once folded to form convenient strengthening ribs. In the patent the possibility of obtaining a cervical collar by suitably shaping the material is also described; however, due to the fixed length of the material strip, fitting such a product to the shape of the patient neck and, especially, chin to obtain a correct and effective immobilization of the neck itself is not possible. Furthermore, the several foldings needed to form the stiffening ribs of the cervical collar along the whole neck circumference make rather slow and complex the use thereof.

An object of the present invention is therefore to put forward an improved cervical collar which can be put away in a limited space.

Another object of the present invention is to put forward a cervical collar having a limited weight and which is easy to carry.

A further object of the present invention is to put forward a cervical collar which is easy to apply and fit to the patient.

These objects are achieved by the present invention through a cervical collar according to claim 1. Further characteristics and advantages of the invention are shown in the respective dependent claims.

In general, a cervical collar comprises a main body able to take at least one first shape condition suitable to allow the storing thereof and at least one second shape condition suitable to be applied to a patient neck to prevent the cervical vertebrae from moving. The collar according to the invention is made in a single piece with a conformable material able to hold the imparted shape, and the main body is shaped to show frontally at least one portion for supporting the patient chin.

Herein and in the following, with the expression "conformable material", a material is meant which can be manually deformed, which is able to hold the imparted shape and which can then be brought back, always manually, to its initial shape.

Therefore, a collar according to the invention can be easily shaped in the use condition: the preset shape provides the collar with a particular easiness in the step of applying it to the patient neck. The collar is easy to store as well due to the possibility of folding the material in the most suitable shape to fill as less space as possible. For example, in the shape condition suitable for the storing, the main body of the collar can be rolled up or, alternatively, folded once or more times to make it as flat as possible.

The material with which the main body is made comprises, for example, an inner foil made of aluminium or its alloys and an outer coating made, for example, with a closed-cells expanded material. The foil made of aluminium alloy aids the folding and the imparted shape to be held, whereas the outer coating made of expanded material, besides acting as a protective coating, is comfortable for the patient.

According to another advantageous aspect of the invention, the portion supporting the chin includes a plurality of conformable fins which allow fitting the collar to the shape of the patient face and neck. The operation of fitting the collar to the patient is therefore made smoother and faster.

However, in order to assure the collar safety after its application, a repositionable closing device is provided, for example a coupling system made of opposite loop and hook strips.

Further characteristics and advantages of the present invention will be more evident from the following description, made by way of example, with reference to the attached drawings, in which:
- Figure 1 is a plan view of a cervical collar according to a possible embodiment of the invention;
- Figure 2 is an axonometric view of the cervical collar of Figure 1 in the shape it takes after the application to a patient neck; and
- Figures 3 and 4 show the schematic and out of scale views of some possible shapes the cervical collar of Figure 1 can take to limit the bulk thereof and allow an easier storing thereof in restricted spaces.

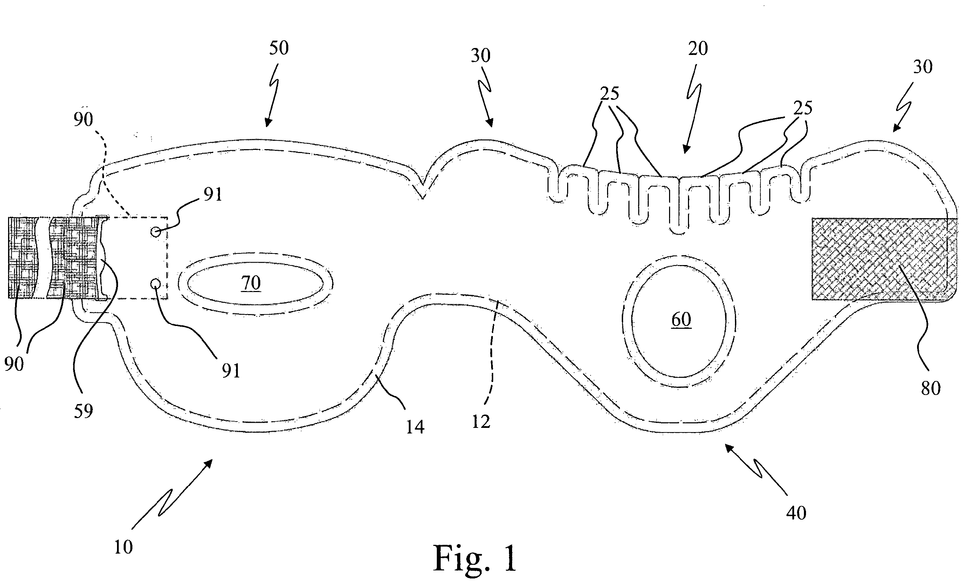

In Figure 1 a cervical collar 10 is shown according to a view taken from the outer face of the collar itself. In this view, the border of an inner foil 12 is pointed out by a dashed line, the foil being made of aluminium or its alloys, whereas the border in solid line shows the coating 14 made of an expanded material, for example an expanded polyurethane, an expanded polyethylene, an expanded elastomer, EVA, combination thereof or the like. The foil 12 and the coating 14 make a single piece which forms the main body of the cervical collar 10.

The foil 12 can have, for example, a thickness of about 0.5 mm, but it can also be realized with thicknesses comprised for example between 0.3 mm and 0.8 mm; thicknesses lower than this range do not provide the collar with enough stiffness, whereas higher thicknesses make the collar shaping mostly difficult.

The thickness of the coating 14 is not particularly relevant and is usually set such that the collar has an overall thickness comprised between 4 mm and 8 mm, for example a thickness of about 5 mm. In any case, a considerable thickness on edges and inner face, that is to say the portions directly contacting the patient, is preferably favoured, whereas on the outer face the coating thickness can also be further reduced.

The main body of the cervical collar 10 is shaped so that to define different functional supporting and/or resting portions for the patient body parts concerned by the collar 10, in particular a portion 20 for the chin support, to which two portions 30 are put side by side for supporting the mandible, a portion 40 for resting the collar 10 to the breastbone and a portion 50 for supporting the nape.

Among the portions 20 and 40 of the collar body 10 an opening 60 is provided to allow the rescue personnel practicing possible tracheotomy interventions also when the collar has already been applied to the patient. A second opening 70 is on the contrary provided in the portion 50 of the body of the collar 10 to aid the nape aeration.

The collar 10 is further provided with a repositionable closing device, for example a loop and hook fastener of the Velcro™ type or the like. The herein depicted closing device comprises, in this case, a plate 80 provided with hooks that is attached, for example by bonding, to the outer face of the body of the collar 10, at the end that is at one of the portions 30 for supporting the mandible. On the contrary, at the opposite end a band 90 is arranged as provided with loops and fixed on the inner face of the body of the collar 10 by means of rivets 91 or the like; the band comes out of the body of the collar 10 through a slot 59 and has a length suitably selected for fitting the collar 10 to the various sizes it is intended for.

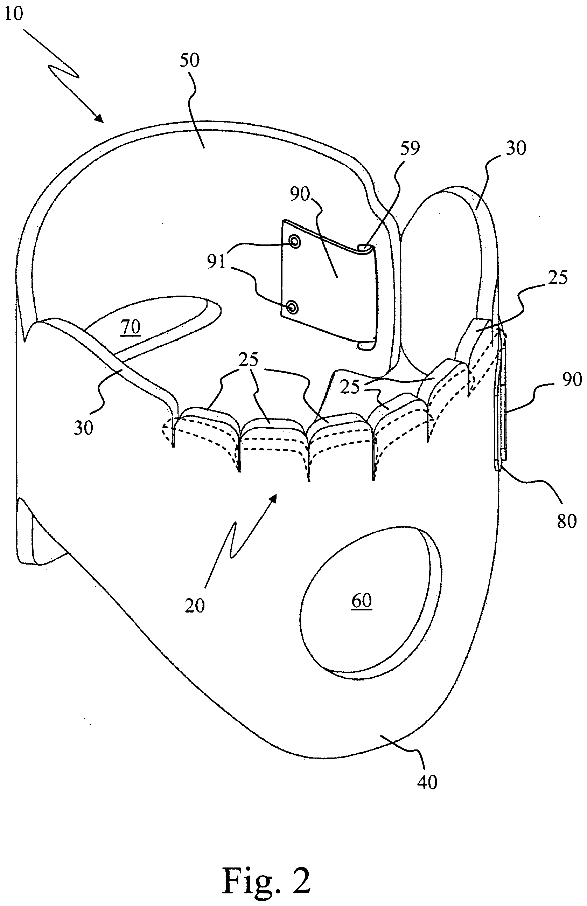

In Figure 2 the cervical collar 10 of Figure 1 is shown in an upright condition, which is the position taken when it is applied to a patient neck. It can be noted that the portion 20 for supporting the chin comprises a plurality of fins 25 which allow simply and rapidly fitting the collar 10 to the shape of the patient face and neck. The fins 25, depicted in Figure 2 by solid line in their original position, can be therefore shaped by imparting a simple folding so that they could take the arrangement represented by the dashed line, for example.

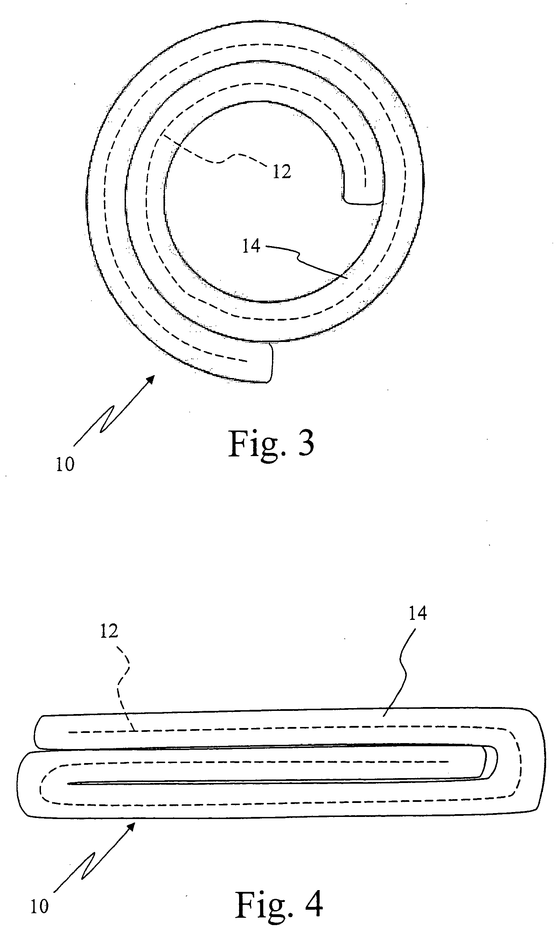

A cervical collar 10 according to the invention is prone to be shaped also in a condition suitable to allow the storing thereof in a reduced space, for example by shaping the main body of the collar 10 in rolled up condition (Figure 3), or in a folded and flattened condition (Figure 4).

In summary, the particular constructive characteristic of a collar 10 according to the invention makes it foldable, able to be rolled up or anyway it allows it taking very limited and compact shape and size suitable for the insertion in a rescuer backpack. Once applied to the patient neck, the collar 10 stiffens up to firmly immobilize him/her. Furthermore, a cervical collar 10 according to the invention is particularly light, about 100 g, and is therefore prone to be adopted in portable first aid kits without weighing particularly on the overall weight thereof.

Various changes can be made by a skilled in the art without departing from the scope of the invention. For example, the closing device can also be different from that herein depicted having loop and hook, as well as the same device can also comprise more than only one plate of hooks 80 and also more than only one band of loops 90.

## Claims

1. A cervical collar (10) comprising a main body able to take at least one first shape condition suitable to allow the storing thereof and at least one second shape condition suitable to be applied to a patient neck to prevent the cervical vertebrae from moving, wherein said main body is made in a single piece with conformable material able to hold the imparted shape, and wherein said main body is shaped for having frontally at least one patient chin supporting portion (20), **characterized in that** said chin supporting portion (20) includes a plurality of conformable fins (25) for fitting the collar (10) to the shape of the patient face and neck.

2. The cervical collar (10) according to claim 1, wherein said main body comprises a core (12) made of aluminium or its alloys and an outer coating (14) made with closed-cells expanded material.

3. The cervical collar (10) according to claim 1, further comprising a repositionable loop and hook closing device (80, 90).

4. The cervical collar (10) according to claim 1 wherein, in said first shape condition suitable to allow the storing thereof, the main body of the collar is rolled up.

5. The cervical collar (10) according to claim 1 wherein, in said first shape condition suitable to allow the storing thereof, the main body of the collar is folded one or more times.

## Patentansprüche

1. Halskragen (10), umfassend einen Hauptkörper, der in der Lage ist, mindestens einen ersten Formzustand anzunehmen, der geeignet ist, dessen Aufbewahrung zu ermöglichen, und mindestens einen zweiten Formzustand anzunehmen, der geeignet ist, an einen Hals eines Patienten angelegt zu werden, um zu verhindern, dass sich die Halswirbel bewegen, wobei der Hauptkörper einstückig mit einem anpassungsfähigen Material hergestellt ist, um die verliehene Form zu halten, und wobei der Hauptkörper so geformt ist, dass er stirnseitig mindestens einen Kinnstützabschnitt (20) für den Patienten aufweist, **dadurch gekennzeichnet, dass** der Kinnstützabschnitt (20) eine Vielzahl von anpassbaren Rippen (25) zum Anpassen des Kragens (10) an die Form des Gesichtes und den Hals des Patienten aufweist.

2. Halskragen (10) nach Anspruch 1, wobei der Hauptkörper einen Kern (12) aus Aluminium oder seinen Legierungen und eine Außenbeschichtung (14) aus geschlossenzelligem expandiertem Material aufweist.

3. Halskragen (10) nach Anspruch 1, ferner umfassend eine repositionierbare Schlaufen- und Hakenverschlussvorrichtung (80, 90).

4. Halskragen (10) nach Anspruch 1, wobei der Hauptkörper des Halskragens in dem ersten Formzustand, der zum Aufbewahren geeignet ist, aufgerollt ist.

5. Halskragen (10) nach Anspruch 1, wobei der Hauptkörper des Halskragens in dem ersten Formzustand, der zum Aufbewahren geeignet ist, ein- oder mehrmals gefaltet ist.

## Revendications

1. Collier cervical (10) comprenant un corps principal capable de d'adopter au moins une première condition de forme appropriée pour permettre son stockage et au moins une seconde condition de forme appropriée pour qu'il soit appliqué sur un cou de patient afin d'empêcher les vertèbres cervicales de bouger, dans lequel le corps principal est fabriqué en une pièce unique à l'aide d'un matériau conformable capable de maintenir la forme imprimée, et dans lequel ledit corps est mis en forme pour avoir frontalement au moins une partie de support de menton de patient (20), **caractérisé en ce que** ladite partie de support de menton (20) inclut une pluralité d'arêtes conformables (25) pour ajuster le collier (10) à la forme du visage et du cou de patient.

2. Collier cervical (10) selon la revendication 1, dans lequel ledit corps principal comprend un noyau (12) constitué d'aluminium ou de ses alliages et un revêtement extérieur (14) constitué d'un matériau expansé à cellules fermées.

3. Collier cervical (10) selon la revendication 1, comprenant en outre un dispositif de fermeture à boucle et crochet repositionnable (80, 90).

4. Collier cervical (10) selon la revendication 1, dans lequel, dans ladite première condition de forme appropriée pour permettre son stockage, le corps principal du collier est enroulé.

5. Collier cervical (10) selon la revendication 1, dans lequel, dans ladite première condition de forme appropriée pour permettre son stockage, le corps principal du collier est plié une ou plusieurs fois.
